# EUROPÄISCHE PATENTSCHRIFT

(11) **EP 4 167 910 B1**
(45) Veröffentlichungstag und Bekanntmachung des Hinweises auf die Patenterteilung: **18.11.2026**
(21) Anmeldenummer: 21739279.4
(22) Anmeldetag: 23.06.2021
(51) Int. Cl.: A61F 2/70, A61F 2/76, H01R 13/629, H01R 13/639

(54) **SCHNITTSTELLENANORDNUNG**
INTERFACE ASSEMBLY
DISPOSITIF D'INTERFACE

(30) Priorität: 23.06.2020 DE 102020116481
(43) Veröffentlichungstag der Anmeldung: 26.04.2023
(73) Patentinhaber: Otto Bock Healthcare Products GmbH, 1110 Wien (AT)
(72) Erfinder: HASLINGER, Martin, 1200 Wien (AT); VUJANIC, Dusan, 1210 Wien (AT)
(74) Vertreter: Gramm, Lins & Partner Patent- und Rechtsanwälte PartGmbB
(86) Internationale Anmeldenummer: PCT/EP2021/067118
(87) Internationale Veröffentlichungsnummer: WO 2021/259985

(56) Entgegenhaltungen:
- EP-B1- 2 219 272
- DE-A1- 102012 013 948
- DE-A1- 102017 131 193
- DE-A1- 102018 107 176
- US-B2- 8 425 620

## Beschreibung

Die Erfindung betrifft eine Schnittstellenanordnung mit einer ersten Schnittstellenkomponente und einer zweiten Schnittstellenkomponente mit einer Schlittenführung zur mechanischen Festlegung der Schnittstellenkomponenten aneinander, wobei an einer der Schnittstellenkomponenten eine von einer Grundfläche hervorstehende Feder und an der anderen Schnittstellenkomponente eine in einer Grundfläche liegende Nut mit korrespondierenden Querschnitten angeordnet sind, wobei die Feder und die Nut die Schnittstellenkomponenten aneinander formschlüssig festlegen und eine Bewegung senkrecht zu den Grundflächen und senkrecht zu der Längserstreckung der Feder und Nut sperren. Insbesondere ist eine solche Schnittstellenanordnung an orthopädietechnischen Einrichtungen vorgesehen, wobei die Schnittstellenkomponenten Teile von Komponenten eines orthopädietechnischen Systems sein können. Die Schnittstellenkomponenten können an einer der jeweiligen Komponenten angeordnet oder ausgebildet sein und ermöglichen eine mechanische Festlegung der beiden Komponenten aneinander. Insbesondere sind die Schnittstellenkomponenten Teil von prothetischen Komponenten, beispielsweise Prothesenhänden, Prothesenfüßen, Prothesenknien. Die Anwendung und der Einsatz der Schnittstellenkomponenten zur Ausbildung der Schnittstellenanordnung ist jedoch nicht auf Prothesen beschränkt, auch Orthesen oder Exoskelette oder andere orthopädietechnische Einrichtungen können eine solche Schnittstellenanordnung aufweisen oder ausbilden.

Orthopädietechnische Systeme bestehen aus mehreren Komponenten, die aneinander angeordnet und zueinander festgelegt werden müssen. Dabei entstehen Schnittstellen an den Übergängen der jeweiligen Komponenten, da die jeweilige orthopädietechnische Einrichtung oder andere Systeme nicht einstückig ausgestaltet werden können oder sollen. Insbesondere zur auswechselbaren Anordnung müssen Schnittstellen vorgesehen sein. Orthesen weisen beispielsweise Gelenke auf, die ein Oberteil mit einem Unterteil gelenkig verbinden. An einem solchen Orthesengelenk sind sogenannte Schienenkästen ausgebildet, die zur Aufnahme der jeweiligen Schiene dienen. Die Schienenkästen sind als eine Nut gefertigt, die eine Schiene an vier Seiten umgibt. Eine Fixierung erfolgt üblicherweise durch Schrauben.

Bei Prothesensystemen erfolgt die Festlegung der Prothesenkomponenten aneinander häufig über einen sogenannten Pyramidenadapter, bei dem eine Komponente eine kalottenförmig nach außen gewölbte Grundfläche aufweist, von der eine mit der Spitze nach unten stehende, vierseitige Pyramide abragt. Die korrespondierende andere Prothesenkomponente weist eine nach innen gewölbte Kalottenfläche mit einer Ausnehmung für den Pyramidenstumpf auf. Seitlich werden Fixierschrauben in Richtung auf die Seitenflächen der Pyramide bewegt, um so eine Einstellung der Komponenten zueinander und deren formschlüssige Festlegung aneinander zu erreichen.

Grundsätzlich ist es auch bekannt, Prothesenkomponenten oder andere orthopädietechnische Komponenten aneinander über eine sogenannte Schlittenführung festzulegen. Dazu ist in einer Komponente eine Feder angeordnet, der eine korrespondierend geformte Nut an der anderen Komponente gegenüberliegt. Zum Verbinden der beiden Komponenten wird die Nut in die Feder eingeschoben, sodass eine Festlegung der beiden Komponenten aneinander erfolgt. Eine Sperrung der Bewegung entgegen der Einführrichtung wird beispielsweise durch Schrauben erreicht, um zu verhindern, dass die Feder ungewollt aus der Nut herausbewegt wird.

Bei immer mehr orthopädietechnischen Systemen kommen elektrische und elektronische Komponenten zum Einsatz, was zu Problemen bei der Durchleitung elektrischer Signale zu den einzelnen Komponenten führt. Die Datenübertragung ebenso wie der Transport ausreichender elektrischer Energiemengen erfordert häufig eine kabelgebundene Verbindung einzelner Komponenten, sodass insbesondere im Bereich der mechanischen Schnittstelle auch eine elektrische Schnittstelle hinzukommt. Diese wird häufig über Stecker oder Schleifkontakte realisiert. Eine solche elektrische Schnittstelle ist anfällig für eine Verschmutzung und birgt die Gefahr, dass die Steckerbestandteile sich voneinander lösen.

Die US 8, 425, 620 B2 betrifft eine Verbindungsanordnung zum lösbaren Sichern eines ersten Segmentes an einem zweiten Segment einer modular aufgebauten künstlichen Gliedmaße. An dem ersten Segment ist ein kraftübertragender Vorsprung in Gestalt einer Schiene angeordnet oder ausgebildet, der in eine Ausnehmung des zweiten Segmentes eingeschoben und darin gesichert werden kann. An dem Vorsprung und innerhalb der Ausnehmung sind elektrische Kontakte angeordnet, sodass eine stabile und lösbare mechanische und elektrische Verbindung zum Aufbau einer modularen künstlichen Gliedmaße bereitgestellt wird.

Die DE 10 2018 107 176 A1 betrifft eine Einrichtung zur mechanischen Anordnung eines Gegenstandes an einem Basiselement mit einem Befestigungselement und einem Einschubteil, das in dem Befestigungselement einführbar ist. Das Einschubteil ist zumindest in einem translatorischen Freiheitsgrad fixiert und weist zumindest einen elektrischen Kontakt auf, der mit einem elektrischen Kontakt des Befestigungselementes in Kontakt steht, wenn das Einschubteil eingeschoben ist. Die elektrischen Kontakte sind an einer Rückwand des Befestigungselementes und einer Stirnseite des Einschubteils angeordnet.

Die DE 10 2017 131 193 A1 betrifft eine Vorrichtung zum Festlegen zweier orthopädietechnische Komponenten mit einem Aufnahmeelement an der ersten Komponente, der in eine Aufnahme der zweiten Komponente einführbar und beispielsweise über Schrauben fixierbar ist. Elektrische Kontakte an dem Aufnahmeelement und in der Aufnahme sind so angeordnet, dass in einem aneinander festgelegten Zustand der orthopädietechnischen Komponenten eine elektrische und/oder Daten übertragende Verbindung ausgebildet wird.

Die EP 2 219 272 B1 betrifft eine Steckvorrichtung mit einem Steckkragen und einem Verriegelungsstift, der um seine Längsachse drehbar gelagert ist und in einer Schließposition in den Steckkragen eingreift. In einer Öffnungsposition liegt eine Ausnehmung des Verriegelungsstiftes in dem Steckkragen, sodass ein Steckerelement gesichert bzw. entnehmbar ist.

Die DE 10 2012 013 948 A1 betrifft eine elektrische Steckerverbinderanordnung mit einem ersten und einem zweiten Steckerverbinderteil, die über Rastelemente aneinander befestigbar sind. Die Steckerverbinderteile bilden einen Steckerkorb, dessen Gehäusemantel durch eine angeformte Bodenplatte abgeschlossen wird, und ein Steckergehäuse, das in den Steckerkorb einführbar ist, aus, so dass das Steckergehäuse im gefügten Zustand zumindest teilweise von dem Steckerkorb umgeben ist. Um ein Eindringen von Spänen zu vermeiden, ist eine Spanschutzwand vorgesehen, die einen engen Freiraum zwischen dem Steckerkorb und dem Steckergehäuse ausbildet, so dass beide Komponenten ohne Dichtung gefügt werden können.

Aufgabe der vorliegenden Erfindung ist es, eine Schnittstellenanordnung bereitzustellen, die die oben beschriebenen Nachteile nicht aufweist. Erfindungsgemäß wird diese Aufgabe durch eine Schnittstellenanordnung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die Schnittstellenanordnung mit einer ersten Schnittstellenkomponente und einer zweiten Schnittstellenkomponente mit einer Schlittenführung zur mechanischen Festlegung der Schnittstellenkomponenten aneinander, wobei an einer der Schnittstellenkomponenten eine von einer Grundfläche hervorstehende Feder und an der anderen Schnittstellenkomponente eine in einer Grundfläche liegende Nut mit korrespondierenden Querschnitten angeordnet sind, wobei die Feder und die Nut die Schnittstellenkomponenten aneinander formschlüssig festlegen und eine Bewegung senkrecht zu den Grundflächen und senkrecht zu der Längserstreckung der Feder und Nut sperren, sieht vor, dass an der Feder und in der Nut elektrische Kontaktelemente angeordnet sind, die in einem gefügten Zustand der Schnittstellenkomponente eine elektrische Verbindung herstellen, wobei die Nut in dem gefügten Zustand mit der Feder stirnseitig abgedichtet ist. Durch die Kombination einer mechanischen Schnittstelle und einer mechanischen Festlegung mehrerer Schnittstellenkomponenten aneinander mit einer elektrischen Schnittstelle durch Kontakte, die an der Nut und in der Feder angeordnet sind, ergibt sich eine abgedichtete, mechanisch festgelegte, elektrische Schnittstelle mit sehr geringem Bauraumbedarf. Gleichzeitig wird sichergestellt, dass bei einer mechanischen Verriegelung der beiden Schnittstellenkomponenten aneinander auch eine elektrische Schnittstelle sicher ausgebildet wird, wodurch unbeabsichtigte Falschkontaktierungen ausgeschlossen werden. Eine Fehlmontage ist nahezu ausgeschlossen und der Montageaufwand wird durch einfaches, schnelles und sicheres Montieren und Demontieren der Schnittstellenpartner auf ein Minimum beschränkt. Eine Verlegung von Kabeln über eine Schnittstelle hinweg ist nicht mehr notwendig, Biegespannungen und Wechselbelastungen an einer Kabelverbindung sind nicht mehr vorhanden.

In einer Weiterbildung der Erfindung ist vorgesehen, dass die elektrischen Kontaktelemente zur Herstellung der elektrischen Verbindung in einer Stirnseite eines Endanschlages der Feder und/oder an einer Stirnseite des Endanschlages der Nut angeordnet sind. Dadurch wird sichergestellt, dass bei einer vollständigen Montage und einem vollständigen Einführen der ersten Schnittstellenkomponente in die zweite Schnittstellenkomponente eine vollautomatische, zwangsgekoppelte und abgedichtete Kontaktverbindung vorliegt. Sind die beiden Schnittstellenkomponenten korrekt mechanisch miteinander verriegelt, liegt auch eine korrekte elektrische Verbindung vor.

Der Endanschlag kann eine Anlagefläche für einen Stecker oder eine Buchse mit den Kontaktelementen ausbilden. Dadurch werden zusätzlich eine mechanische Verriegelung und ein Schutz der elektrischen Komponenten bewirkt, wenn die Steckerkomponenten in die Buchsenkomponenten eingeführt werden, sobald die Nut und die Feder ineinander hineingeschoben sind.

Erfindungsgemäß ist vorgesehen, dass die Nut in dem gefügten Zustand mit der Feder stirnseitig abgedichtet ist, beispielsweise durch eine an dem hinteren und/oder vorderen Ende angeordnete Dichtung an der Feder, durch einen Vorsprung oder einen Endanschlag, gegebenenfalls mit einer Dichtung oder durch Anordnung einer entsprechenden Dichtung in der Nutwandung. Durch die abgedichtete Nut wird weiterhin das Eindringen von Schmutz und Feuchtigkeit in die mechanische Verbindung und dadurch auch in die elektrische Kontaktierung verhindert oder zumindest vermindert, sodass eine dauerhaft zuverlässige Kontaktierung bereitgestellt werden kann.

Vorteilhafterweise führt eine Kabelverbindung von den elektrischen Kontaktelementen von der Feder und der Nut weg durch die jeweiligen Schnittstellenkomponenten, sodass keine Relativbewegung im Bereich der elektrischen Kontaktierung zwischen den einzelnen Kabeln oder Kabelzuleitungen erfolgt. Dies verhindert eine komplizierte Verlegung von Kabeln außerhalb der Schnittstellenkomponenten und stellt sicher, dass die Kabel korrekt verlegt sind.

In zumindest einer der Schnittstellenkomponente kann eine Kabeldurchführung und/oder eine Steckeraufnahme angeordnet oder ausgebildet sein, um die Kabel entsprechend innerhalb der Schnittstellenkomponente zu führen bzw. um Stecker aufzunehmen, damit eine elektrische Kontaktierung stattfinden kann. Die elektrischen Kontaktelemente sind beispielsweise in einem Stecker ausgebildet, der separat ausgebildet und in der Steckeraufnahme in der Schnittstellenkomponente eingeführt und festgelegt werden kann.

Die Schnittstellenkomponenten weisen eine Verriegelungseinrichtung auf, die eine Verlagerung der Schnittstellenkomponente zueinander entlang der Längserstreckung der Schlittenführung sperrt. Durch die Anordnung der Verriegelungseinrichtung an den Schnittstellenkomponenten wird die Integration auch einer mechanischen Festlegung in die jeweilige Schnittstellenkomponente ermöglicht. Zur Montage der einzelnen Schnittstellenkomponenten aneinander werden dann keine separaten Bauteile mehr benötigt, da die Bestandteile der Verriegelungseinrichtung an den Schnittstellenkomponenten angeordnet sind.

Die Verriegelungseinrichtung weist zwei Formschlusskomponenten auf, die wiederholbar in und außer Eingriff miteinander bringbar sind. Dadurch können die einzelnen Komponenten wiederholt aneinander festgelegt und voneinander getrennt werden. Eine der beiden Formschlusskomponenten ist drehbar oder schwenkbar an oder in einer Schnittstellenkomponente gelagert und weist zumindest eine Abflachung, einen unrunden Querschnitt oder eine Ausnehmung auf. Durch die Verdrehbarkeit oder Verschwenkbarkeit der Formschlusskomponente ist es möglich, neben einer einfachen Verriegelung und Sperrung der Beweglichkeit entgegen der Einführrichtung eine Verspannung der Schlittenführung einander zu bewirken. Die einzelnen Seitenflächen und Anlageflächen der Schlittenführung werden gegeneinander verspannt, sodass sich ein sicherer Halt durch eine formschlüssige Verriegelung und zudem ein Spielausgleich durch eine axiale oder radiale Verspannung der Fügeflächen der Schlittenführung ergibt. Dadurch ist es möglich, die einzelnen Bauteile der Schlittenführung weniger präzise fertigen zu müssen, die Gesamtkonstruktion ist fehlertoleranter, weist einen geringen Komplexitätsgrad auf und ermöglicht eine günstige Fertigung der gesamten Schnittstellenstellenanordnung.

Die Formschlusskomponente verspannt die Schnittstellenkomponenten in der Verriegelungsstellung gegeneinander, bewirkt also eine klemmende Verbindung zwischen den einzelnen Schnittstellenkomponenten.

Einer der Formschlusskomponenten kann ein Sicherungselement zugeordnet sein, das von einer Stirnseite der Schnittstellenkomponente aus zugänglich ist. Dadurch ist es möglich, die Montage und Verriegelung der Schnittstellenkomponenten in nur einer Fügerichtung durchzuführen, sodass auch bei wenig Bauraum und schlechter Zugänglichkeit, was bei orthopädietechnischen Einrichtungen häufiger der Fall ist, eine einfache und schnelle sowie sichere Verriegelung der Schnittstellenkomponenten aneinander möglich ist.

Das Sicherungselement der Formschlusskomponente kann formschlüssig in das Formschlusselement eingreifen, beispielsweise in eine Ausnehmung einrasten oder in eine Ausnehmung eingelegt werden. Das Sicherungselement kann alternativ oder zusätzlich gegen das Formschlusselement vorgespannt sein oder über eine Verzahnung mit dem Formschlusselement gekoppelt werden. Die Verzahnung ist vorteilhafterweise selbsthemmend ausgebildet, sodass nur durch eine entsprechende Betätigung des Sicherungselementes eine Verlagerung und damit eine Freigabe bzw. Verriegelung des Formschlusselementes in der verriegelten oder entriegelten Stellung möglich ist.

Eine der Formschlusskomponenten kann als Nut ausgebildet sein, wodurch eine große Oberfläche bereitgestellt wird, mit der die andere Formschlusskomponente wechselwirken kann. Dadurch verringert sich die Flächenpressung, wodurch die einzelnen Formschlusskomponenten vergleichsweise klein ausgebildet werden können. Dies vergrößert den Spielraum für Designlösungen und verringert den notwendigen Bauraum.

Die erste Schnittstellenkomponente kann als eine distale Abschlussplatte eines Prothesenschaftes oder einer Prothesenkomponente ausgebildet sein, die zweite Schnittstellenkomponente kann als proximale Abschlusskomponente einer Prothesenhand oder einer prothetischen Greifeinrichtung ausgebildet sein.

Nachfolgend werden Ausführungsbeispiele der Erfindung anhand der beigefügten Figuren näher erläutert. Es zeigen:
- Figur 1 -: eine perspektivische Ansicht einer Schnittstellenanordnung mit zwei Schnittstellenkomponenten in nicht gefügtem Zustand;
- Figur 2-: eine Explosionsdarstellung der Komponenten der Figur 1;
- Figur 3 -: eine Untenansicht der Darstellung der Figur 2;
- Figur 4 -: eine Schrägdraufsicht auf die Schnittstellenanordnung im ungefügten Zustand gemäß Figur 1;
- Figur 5 -: eine Untenansicht der Stellung gemäß Figur 4;
- Figur 6 -: eine Darstellung der Schnittstellenanordnung mit gefügten Schnittstellenkomponenten;
- Figur 7 -: eine alternative Ausführungsform im gefügten Zustand;
- Figur 8 -: Figur 7 in nicht gefügtem Zustand;
- Figur 9 -: eine Explosionsdarstellung der Komponenten der Ausführungsform gemäß Figur 8;
- Figur 10 -: eine Untenansicht der Figur 9;
- Figur 11 -: eine Untenansicht der Schnittstellenkomponenten;
- Figur 12 -: eine andere Ansicht der Figur 8;
- Figur 13 -: eine teiltransparente Darstellung im montierten Zustand;
- Figur 14 -: ein Anwendungsbeispiel der Erfindung an einer Prothesenhand; sowie
- Figur 15 -: ein Anwendungsbeispiel der Erfindung an einem Prothesenbein.

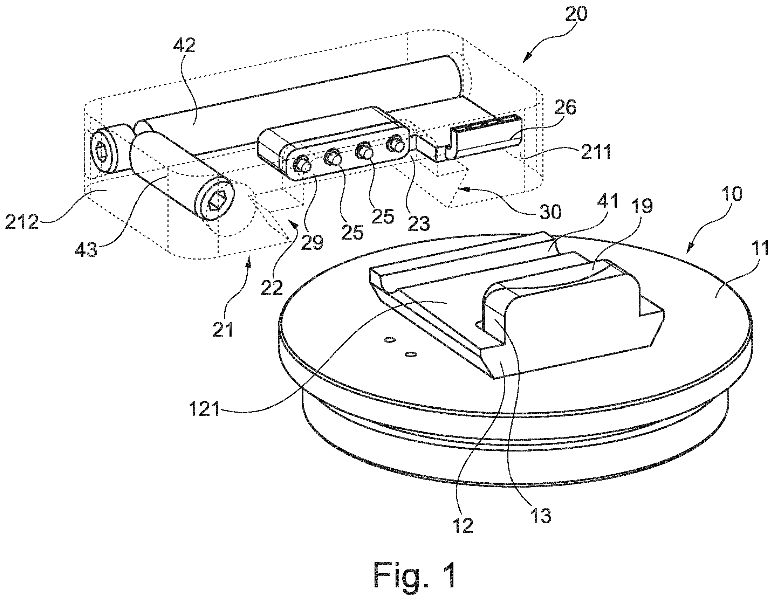

In der Figur 1 ist in einer perspektivischen Ansicht eine Schnittstellenanordnung mit einer ersten Schnittstellenkomponente 10 und einer zweiten Schnittstellenkomponente 20 gezeigt. Die zweite Schnittstellenkomponente 20 ist nur in durchbrochenen Linien in der Umrissdarstellung gezeigt, um die sonst nicht sichtbaren Teile der zweiten Schnittstellenkomponente 20 darzustellen. Die erste Schnittstellenkomponente 10 weist eine Grundfläche 11 auf, von der eine Feder 12 absteht. Ist die erste Schnittstellenkomponente 10 als eine proximale Komponente ausgebildet, erstreckt sich die Feder 12 von der Grundfläche 11 in distale Richtung, wenn es sich sich bei der Schnittstellenanordnung um eine Verbindung im Rahmen der Orthopädietechnik handelt. Die Feder 12 ist in Gestalt einer abgewandelten Schwalbenschwanzfeder ausgebildet und weist einen nach oben stehenden Endanschlag 13 auf, der über eine Federoberfläche 121, die im Wesentlichen parallel zur Grundfläche 11 orientiert angeordnet ist, hervorsteht. Innerhalb der Federoberfläche 121 ist eine quer zur Längserstreckung der Feder 12 verlaufende Formschlusskomponente 41 in Gestalt einer Ausnehmung, Nut oder Auskehlung ausgebildet. Die Formschlusskomponente 41 ist innerhalb der Federoberfläche 121 angeordnet, die Federoberfläche 121 erstreckt sich somit beiderseits der Längserstreckung der Feder 12 von der Formschlusskomponente 41. Innerhalb der Feder 12 ist ein Stecker 19 angeordnet, der sich zwischen dem Endanschlag 13 und der Formschlusskomponente 41 befindet. Der Stecker 19 liegt an dem Endanschlag 13 an und ragt über die Federoberfläche 121, jedoch nicht über den Endanschlag 13 hinaus.

Die zweite Schnittstellenanordnung 20, die beispielsweise an einer distalen Komponente einer orthopädietechnischen Einrichtung angeordnet oder ausgebildet sein kann, weist eine Nut 22 auf, die in ihrer Form korrespondierend zu der Feder 12 ausgebildet ist. Die Nut 22 erstreckt sich von einer Stirnfläche 212 weg in die zweite Schnittstellenkomponente 20 hinein. An der Unterseite ist eine Grundfläche 21 ausgebildet, von der aus sich die Nut 22 nach oben hin erstreckt. Die Grundfläche 21 der zweiten Schnittstellenkomponente 20 ist vorteilhafterweise so ausgebildet, dass sie auf der Grundfläche 11 der ersten Schnittstellenkomponente 10 aufliegt oder an dieser anliegt, zumindest einen nur geringen Spalt dazwischen ausbildet. Innerhalb der Nut 22 an einer der Stirnfläche 211 abgewandten Ende der Nut 22 ist ein Stecker 29 mit elektrischen Kontakten 25 angeordnet. Der Stecker 29 schließt im Wesentlichen bündig mit einem Endanschlag 23 ab, die elektrischen Kontakte 25 oder Steckerelemente ragen über den Endanschlag 23 vorteilhafterweise hinaus.

Weiterhin sind in der zweiten Schnittstellenkomponente 20 Bohrungen angeordnet, die sich senkrecht von der Stirnfläche 211 entlang der Längserstreckung der Nut 22 sowie rechtwinklig dazu erstrecken. In der quer zur Längserstreckung der Nut 22 angeordneten Bohrung ist ein zweites Formschlusselement 42 angeordnet, das eine formschlüssige Verriegelung mit der ersten Formschlusskomponente 41 in der Feder 12 ermöglichen kann. Die zweite Formschlusskomponente 42 wird später näher erläutert und ist als Zylinder mit einer Abflachung oder als eine unrunde Komponente angeordnet, die über eine Öffnung in einer Seitenfläche 212 beispielsweise über einen Schraubendreher, einen Sechskantschlüssel oder ein ähnliches Werkzeug bewegt werden kann. Durch eine Drehung um die Längserstreckung der drehbar gelagerten zweiten Formschlusskomponente 42 ist es möglich, eine Verriegelung und Entriegelung im gefügten Zustand zu ermöglichen. Die verriegelte Stellung oder auch die entriegelte Stellung kann beispielsweise über ein Sicherungselement 43 in Gestalt einer Madenschraube oder einer anderen Verriegelungseinrichtung fixiert werden. Das Sicherungselement 43 weist eine Zugangsöffnung auf, die innerhalb der Stirnfläche 212 ausgebildet ist, sodass eine Sicherung und gegebenenfalls auch Betätigung über das Sicherungselement 43 erfolgen kann. Dazu sind beispielsweise das Sicherungselement 43 und die zweite Formschlusskomponente 42 über ein Gewinde oder eine Verzahnung miteinander gekoppelt, sodass durch Drehung des Sicherungselementes 43 eine Drehung der zweiten Formschlusskomponente 42 innerhalb der zweiten Schnittstellenkomponente 20 erfolgen kann. Durch eine umgekehrte Drehrichtung erfolgt eine entsprechende Entriegelung. Die Sicherung in der jeweiligen Stellung kann beispielsweise durch ein selbsthemmendes Gewinde innerhalb der Bohrung oder Ausnehmung und an der Außenseite des Sicherungselementes 43 erreicht werden.

Die Nut 22 zusammen mit der Feder 12 bilden die Schlittenführung 30 zwischen der ersten Schnittstellenkomponente 10 und der zweiten Schnittstellenkomponente 20. Dabei werden nach dem Einführen der Feder 12 in die Nut 22 alle rotatorischen Freiheitsgrade sowie zwei translatorische Freiheitsgrade gesperrt, bis der jeweilige Endanschlag 13, 23 erreicht ist. Nach Erreichen der jeweiligen Endanschläge oder der Endposition sind die elektrischen Kontakte der Stecker 29, 19 sicher kontaktiert und die Formschlusselemente 41, 42 der dadurch ausgebildeten Verriegelungseinrichtung können miteinander in Eingriff gebracht werden. Die jeweiligen Steckerkomponenten oder Buchsenkomponenten der elektrischen Kontakte 25 sind mit Kabelverbindungen gekoppelt, die in der Darstellung der Figur 1 nur an der zweiten Schnittstellenkomponente 20 in der Kabelverbindung 26 zu erkennen sind. Die Kabelverbindung 26 führt zu weiteren elektrischen oder elektronischen Komponenten an der jeweiligen Einrichtung, die mit der Schnittstellenanordnung oder Schnittstellenkomponente 20 verbunden ist.

Neben einer Ausgestaltung der elektrischen Kontakte 25 als hervorstehende Stecker können diese auch als Buchsen oder flächige Elemente ausgebildet sein, die in der gefügten Stellung mit den korrespondierenden elektrischen Kontakten der ersten Schnittstellenkomponente 10 in Kontakt tritt. Die elektrischen Kontakte 25 können federnd gelagert sein, um eine sichere Kontaktierung auch vor Erreichen der Endanschläge und einer mechanischen Verriegelung über die Formschlusskomponenten 41, 42 zu erreichen.

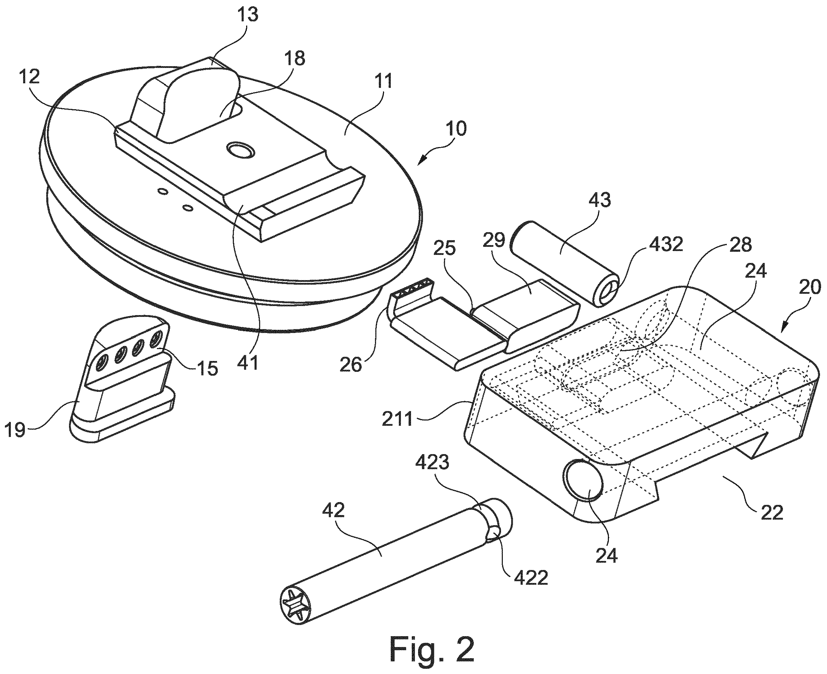

In der Figur 2 sind die einzelnen Elemente der Schnittstellenkomponente 10, 20 dargestellt. Die erste Schnittstellenkomponente 10 mit der über die Grundfläche 11 hinausstehenden Feder mit einem Schwalbenschwanzprofil weist eine Steckeraufnahme 18 auf, die durch den Grundkörper und die Grundfläche 11 sowie die Feder 12 hindurchgeht. In dieser Steckeraufnahme 18 wird der Stecker 19 mit den elektrischen Kontakten 15 eingesetzt oder eingeschoben, im dargestellten Ausführungsbeispiel von der der Grundfläche 11 abgewandten Unterseite. Durch den Absatz an dem Stecker 19 wird gewährleistet, dass die Oberkante des Steckers 19 nicht über die Oberkante des Endanschlages 13 hinausragt und eine sichere Verriegelung und Festlegung an der ersten Schnittstellenkomponente 10 erfolgen kann. Der Stecker 19 liegt vollflächig mit der Rückseite, also der dem elektrischen Kontakten 15 abgewandten Seite, an dem Endanschlag 13 an. In dem dargestellten Ausführungsbeispiel ist die Rückseite des Steckers 15 gerundet ausgebildet, es können jedoch auch andere Formgebungen möglich sein. Die Steckeraufnahme 18 dient zur Festlegung des Steckers 19 bzw. der Steckerkomponente 19 mit den elektrischen Kontakten 15 und ermöglicht die Aufnahme der mechanischen Kontaktierungskräfte und stellt ein Widerlager bereit.

Die zweite Schnittstellenkomponente 20 weist, wie bereits anhand der Figur 1 erläutert, neben der von der Stirnseite 211 beginnenden, durchgehenden Nut 22 an der Unterseite oder Grundfläche 21 zwei Bohrungen 24 auf, die einmal zur Aufnahme des zweiten Formschlusselementes 42 und einmal zur Aufnahme des Sicherungselementes 43 dienen. Beide Bohrungen 24 schneiden sich, sodass die zweite Formschlusskomponente 42 mit dem Sicherungselement 43 in Kontakt treten kann. An einem Ende der zweiten Formschlusskomponente 42 ist eine umlaufende Nut 423 ausgebildet, die eine Bohrung oder Ausnehmung 422 aufweist. Es können mehrere, über den Umfang verteilte Bohrungen oder Ausnehmungen 422 im Umfang der Nut 423 ausgebildet sein. An dem der zweiten Formschlusskomponente zugewandten Ende des Sicherungselementes 43 ist beispielsweise eine federnd gelagerte Kugel oder ein anderes Eingriffselement gelagert, die oder das in Richtung auf die zweite Formschlusskomponente 42 vorgespannt ist und in die jeweiligen Bohrungen oder Ausnehmungen 422 eingreift, wenn eine entsprechende Ausrichtung der zweiten Formschlusskomponente 42 vorhanden ist. Dadurch wird die zweite Formschlusskomponente gegen ein Verdrehen innerhalb der Bohrung 24 gesichert. Alternativ dazu kann an dem Ende des Sicherungselementes 43 eine Verzahnung 432 ausgebildet sein, die in eine korrespondierende Verzahnung 422 innerhalb der Nut 423 eingreift.

Die zweite Steckerkomponente 29 mit den elektrischen Kontakten 25 wird in eine Ausnehmung oder Aufnahme 28 innerhalb der zweiten Schnittstellenkomponente 20 eingeschoben. Die Steckeraufnahme 28 ermöglicht eine formschlüssige Festlegung des Steckers 29 sowie der Kabelführung mit der Kabelverbindung 26 innerhalb der zweiten Schnittstellenkomponente 20.

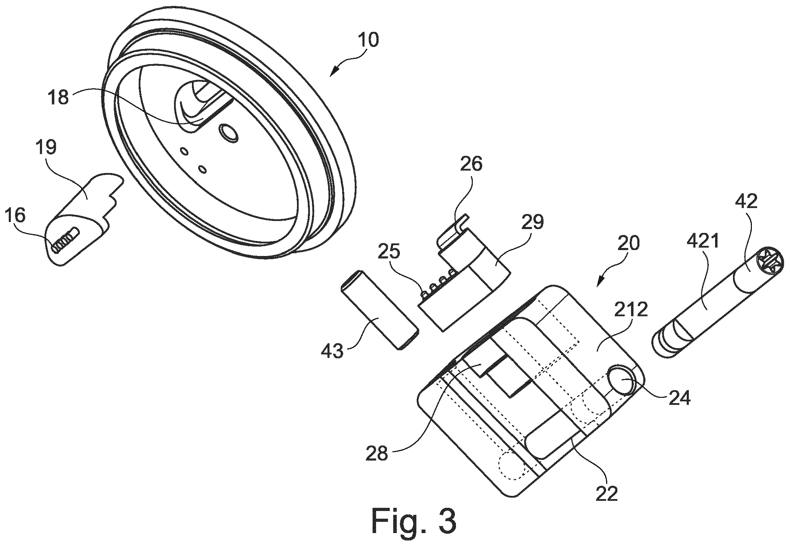

In der Figur 3 ist eine Untenansicht der Darstellung gemäß Figur 2 gezeigt, der zu entnehmen ist, dass die Steckerdurchführung 18 für den Stecker 19 durchgängig durch die erste Schnittstellenkomponente 10 ausgebildet ist. An der Unterseite des Steckers 19 für die erste Schnittstellenkomponente 10 ist die entsprechende Kabelverbindung 16 gezeigt. An dem Stecker 29 der zweiten Schnittstellenkomponente 20 sind sowohl das Gehäuse als auch die hervorstehenden elektrischen Kontakte 25 zu erkennen. An der Oberseite der Nut 22 sind die Ausnehmungen für die Steckeraufnahme 28 zu erkennen, ebenso wie die quer verlaufende Bohrung 24, die sich in dem dargestellten Ausführungsbeispiel über die gesamte Breite, also senkrecht zu einer Seitenfläche 212 und senkrecht zu der Längserstreckung der Nut 22 erstreckt. Der Bohrungsdurchmesser ragt in die Nut 22 hinein, sodass bei einer Einbringung eines zylindrischen Körpers in die Bohrung 24 die Feder 12 nicht vollständig bis zum Ende der Nut 22 oder bis zum Kontakt der beiden Endanschläge eingeführt werden könnte. Um dies zu ermöglichen, ist an der zweiten Formschlusskomponente 42 eine Abflachung 421 ausgebildet, die so bemessen ist, dass sie bündig mit der Oberseite der Nut 22 oder dem Nutgrund abschließt, zumindest jedoch so weit zurücksteht, dass die Federoberseite 121 darunter entlangbewegt werden kann.

Ist die Feder 12 vollständig in die Nut 22 eingeführt, liegt die Ausnehmung oder erste Formschlusskomponente 41 an der Oberseite der Feder 12 direkt unterhalb der Bohrung 24 und unterhalb der zweiten Formschlusskomponente 42. Die zweite Formschlusskomponente 42 kann verdreht werden, sodass der abgerundete, zylindrische Teil der Oberfläche der zweiten Formschlusskomponente 42 in die korrespondierende Nut 41 eingreifen kann. Bevorzugt füllt die zweite Formschlusskomponente 42 die erste Formschlusskomponente 41 in dem Endzustand oder dem vollständig gefügten Zustand der beiden Schnittstellenkomponenten aus. Ist zu Beginn der Drehbewegung der zweiten Formschlusskomponente die Feder 12 noch nicht vollständig in der Nut 22 eingeschoben, wird durch die Verdrehbewegung dieses nachgeholt und die beiden Schnittstellenkomponenten 10, 20 in die gewünschte und vorgegebene Endstellung bewegt. Durch Einrasten einer vorgespannten Kugel oder eines Eingriffselementes oder durch eine entsprechende Verdrehung des Sicherungselementes 43 wird dann die eingenommene Stellung fixiert, sodass auch der dritte translatorische Freiheitsgrad gesperrt ist.

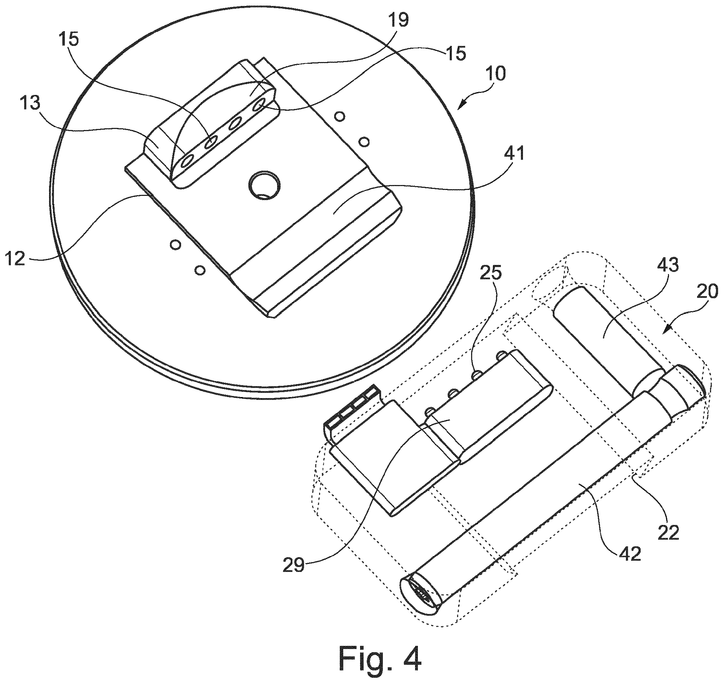

Figur 4 zeigt die einzelnen Komponenten in eine perspektivische Schrägdraufsicht, wobei die Elemente der Schnittstellenkomponenten 10 und 20 bereits gefügt sind. Der Stecker 29 mit den elektrischen Kontakten 25 ist innerhalb der zweiten Schnittstellenkomponente 20 innerhalb der Aufnahme 28 eingeführt. Die zweite Formschlusskomponente 42 befindet sich innerhalb der entsprechenden Bohrung 24, das Sicherungselement 43 steht in Eingriff mit der zweiten Formschlusskomponente 42, wobei der zylindrische Abschnitt der zweiten Formschlusskomponente 42 nach oben gedreht ist, sodass die Abflachung 421, die nicht dargestellt ist, vorteilhafterweise bündig mit der Oberseite oder dem Nutgrund der Nut 22 abschließt.

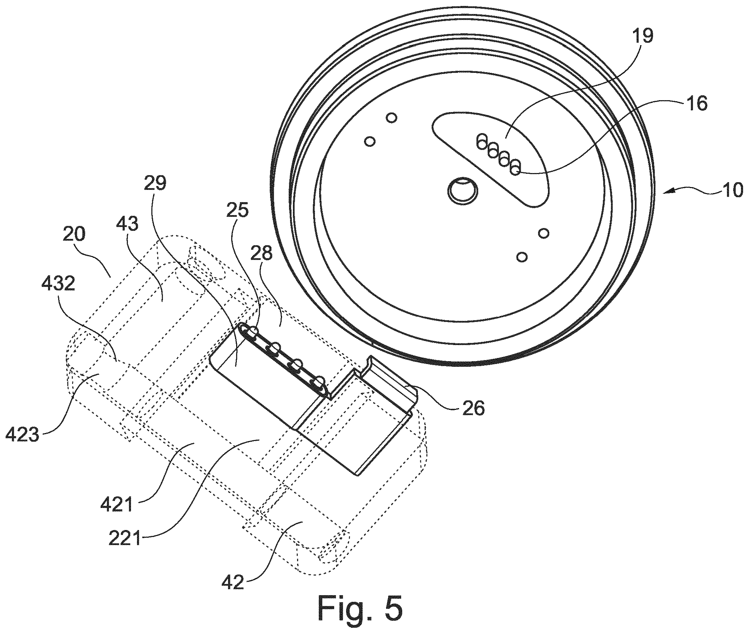

Die Untenansicht der Figur 4 ist in der Figur 5 dargestellt, wobei die einzelnen Elemente der zweiten Schnittstellenkomponente 20 nur teilweise dargestellt sind. Die abgeflachte Unterseite 421, die bündig mit der Nutoberseite oder dem Nutgrund 221 abschließt, ist ebenso zu erkennen wie die Anordnung des Steckers 29 in der Steckeraufnahme 28. Das Sicherungselement 43 greift über ein Gewinde 432 in ein korrespondierendes Gewinde 423 ein oder über eine vorgespannte Kugel in eine entsprechende Ausnehmung. Alternativ kann durch Einschrauben des Sicherungselementes in Gestalt einer Madenschraube eine klemmende und sichere Fixierung der zweiten Formschlusskomponente 42 in der jeweils gewünschten Stellung erreicht werden. Der Stecker 19 schließt bündig mit der Unterseite der Grundfläche 11 der ersten Schnittstellenkomponente 10 ab und die Kabelverbindung 16 ragt nach unten durch die Schnittstellenkomponente 10 hindurch.

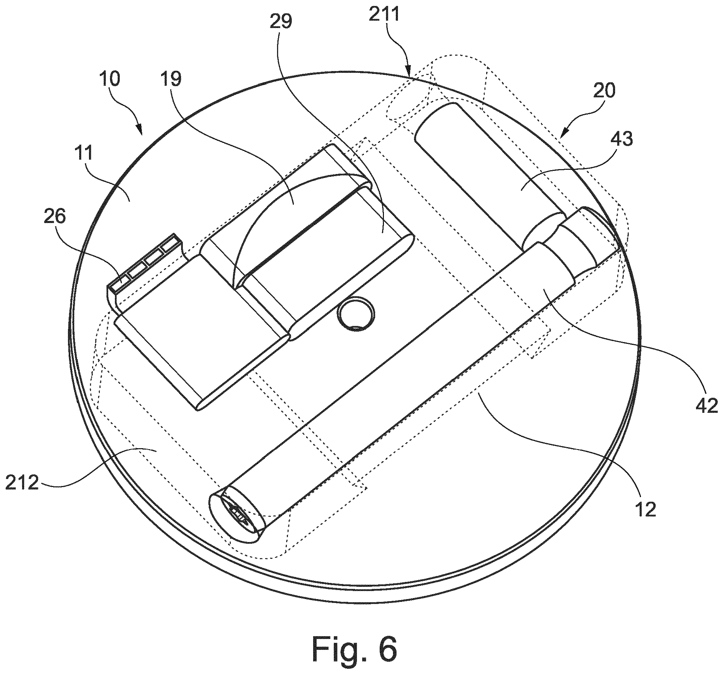

In der Figur 6 ist die Endstellung gezeigt, wenn die beiden Schnittstellenkomponenten 10, 20 gefügt sind. Zur besseren Übersichtlichkeit ist die zweite Schnittstellenkomponente 20 in unterbrochenen Linien gezeigt. Es ist zu erkennen, dass in dem endgefügten Zustand die beiden Stecker 19, 29 mit ihren Stirnflächen aneinander anliegen, somit sind die Endanschläge der Schlittenführung aus Nut 22 und Feder 12 erreicht. Von den elektrischen Kontakten der Steckerelemente 19, 29 führt die Kabelverbindung 26 nach oben von der Grundfläche 11 weg. Auf der anderen Seite wird durch den Stecker 19 hindurch die Kabelverbindung 16 in entgegengesetzte Richtung geführt. Das Formschlusselement 42 befindet sich in formschlüssigem Eingriff mit der Nut in der Feder 12, das Sicherungselement 43 verriegelt die zweite Formschlusskomponente, wobei die Zugänglichkeit von der Stirnseite 211 in die Bohrung 24 hinein gegeben ist. Wird die zweite Formschlusskomponente 42 durch das Sicherungselement 43 bewegt, kann eine Aktuierung und Sicherung von der Stirnseite 211 aus erfolgen, sodass eine Montage und Fügebewegung in einer Orientierung erfolgen kann, sodass die Zugänglichkeit nur von der Stirnseite, also in der Verschieberichtung der Nut 22 und Feder 12 erfolgen kann. Grundsätzlich ist jedoch auch die Zugänglichkeit über eine Seitenfläche 212 möglich und vorgesehen.

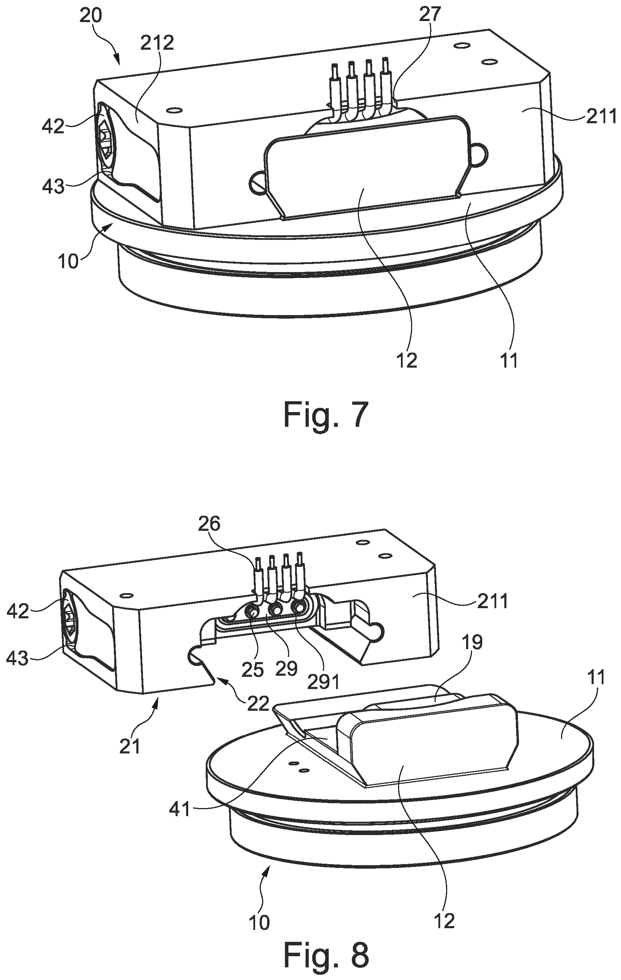

Eine Variante der Erfindung ist in den Figuren 7 bis 13 gezeigt. Gleiche Bezugszeichen bezeichnen gleiche Bauelemente. Der grundsätzliche Aufbau der Schnittstellenkomponenten 10, 20 ist unverändert, sodass nur auf Abweichungen oder Besonderheiten eingegangen wird. In der Figur 7 ist die gefügte Stellung der beiden Schnittstellenkomponenten 10, 20 gezeigt. Die Unterseite oder Grundfläche der zweiten Schnittstellenkomponente 20 liegt auf der Grundfläche 11 der ersten Schnittstellenkomponente 10 auf. Die Feder 12, die von der Grundfläche 11 nach oben absteht, ist vollständig in die Nut in der zweiten Schnittstellenkomponente 20 eingeführt. Die Rückseite der Feder 12 schließt mit der Stirnseite 211 der zweiten Schnittstellenkomponente 20 ab. An der Oberseite der zweiten Schnittstellenkomponente 20 ist eine Kabeldurchführung 27 ausgebildet, die mittig oberhalb des Endanschlages 13 der Feder 12 ausgebildet ist. Rechts und links neben der Feder 12 sind Bohrungen oder Ausnehmungen innerhalb des Grundkörpers der zweiten Schnittstellenkomponente 20 ausgebildet, um das Einführen der Feder 12 in die Nut 22 zu erleichtern.

Abweichend von der ersten Ausführungsform ist in der Figur 7 das Sicherungselement 43 nicht von der Stirnseite 211 zugänglich, sondern an einer Seitenfläche 212 angeordnet. An der zweiten Formschlusskomponente 42 ist ein Vorsprung angeordnet oder ausgebildet, der eine Verdrehung entgegen dem Uhrzeigersinn aus der vorliegenden Stellung verhindert. Der Vorsprung liegt an einem Anschlag an, der an der zweiten Schnittstellenkomponente 20 ausgebildet ist. Das Sicherungselement 43 liegt an der dem Anschlag gegenüberliegenden Seite an dem Vorsprung an und sichert somit die zweite Formschlusskomponente 42 gegen ein Verdrehen in Uhrzeigersinn. Das Sicherungselement 43 kann federnd in Richtung auf die zweite Schnittstellenkomponente 20 vorgespannt gelagert sein, um eine dauerhafte Sicherung zu gewährleisten. Zum Herstellen der formschlüssigen Verriegelung der beiden Schnittstellenkomponenten 10, 20 aneinander muss dann das Sicherungselement 43 entgegen einer Federkraft aus der dargestellten Verriegelungsstellung in eine Entriegelungsstellung gebracht und anschließend die zweite Formschlusskomponente 42 im Uhrzeigersinn gedreht werden, damit die oben bereits beschriebene formschlüssige Verriegelung erreicht werden kann. Nachdem der Vorsprung unten an einem Anschlag anliegt, ist die vollständige Verriegelung erreicht und das Sicherungselement 43 kann wieder in die Ausnehmung eingelegt werden. Zum Lösen der Verbindung erfolgt eine umgekehrte Bewegung. Erst dann ist es möglich, die Schnittstellenkomponente 20 von den Endanschlägen weg entlang der Schlittenführung von der ersten Schnittstellenkomponente 10 weg zu schieben und die formschlüssige Verriegelung zu lösen. Mit der Lösung der beiden Schnittstellenkomponenten wird gleichzeitig auch die elektrische Kontaktierung gelöst.

Der gelöste, nicht mehr gefügte Zustand der beiden Schnittstellenkomponenten 10, 20 ist in der Figur 8 gezeigt. Die beiden Steckerkomponenten 19, 20 sind ebenso zu erkennen wie die elektrischen Kontakte 25 an der zweiten Schnittstellenkomponente 20 sowie eine umlaufende Dichtung 291, die um die elektrischen Kontakte 25 herum angeordnet ist. Befindet sich die Schnittstellenanordnung in dem gefügten Zustand gemäß Figur 7, liegt die Dichtung 291 an einer Stirnfläche des korrespondierenden Steckers oder Steckerelementes 19 an und gewährleistet eine Abdichtung, sodass Schmutz und Feuchtigkeit nicht an die elektrische Kontaktierung gelangen kann.

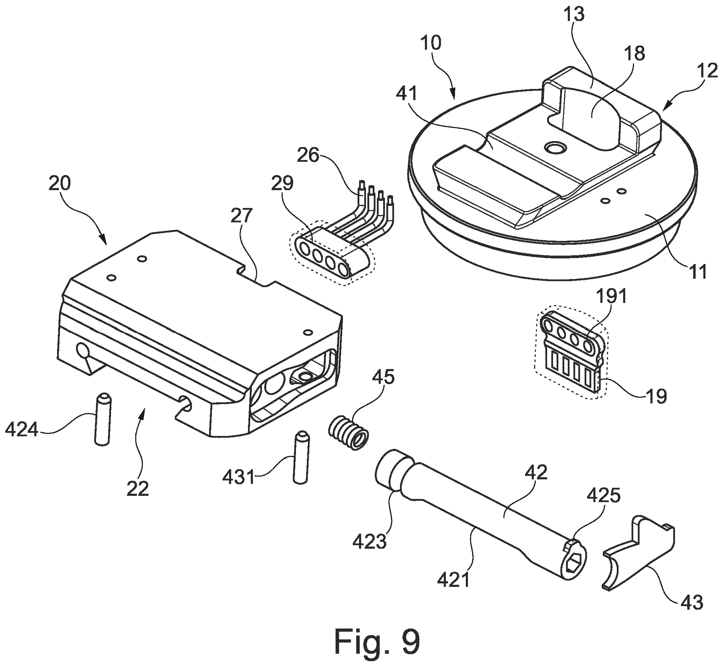

In der Figur 9 sind die einzelnen Komponenten in einer Explosionsdarstellung gezeigt. Der Aufbau der ersten Schnittstellenkomponente 10 entspricht der der Ausführungsform gemäß der Figuren 1 bis 6. An der Steckerkomponente 19 ist ebenfalls eine Dichtung 191 angeordnet, die Dichtungen 291, 191 können aneinander anliegen oder eine Doppeldichtung ausbilden, bei der eine Dichtung die andere Dichtung außen umgibt. Dadurch wird ein verbesserter Dichteffekt erzielt.

An der zweiten Schnittstellenkomponente 20 ist die Kabeldurchführung 27 als Ausnehmung an der Oberseite zu erkennen. Ebenfalls ist die durchgängige Nut 22 in Schwalbenschwanzform sowie die in dem Grundkörper der zweiten Schnittstellenkomponente 20 angeordneten Querbohrungen zu erkennen. Die zweite Formschlusskomponente 42 weist an der Unterseite die Abflachung 421 auf, an einem Ende ist der Vorsprung 425 zu erkennen, der in dem dargestellten Ausführungsbeispiel so orientiert ist, dass er in der dargestellten Stellung eine Entriegelung und Verlagerbarkeit der beiden Schnittstellenkomponenten 10, 20 zueinander erlaubt. Wird die zweite Formschlusskomponente 42 um 180° gedreht, liegt der Vorsprung 425 unten an einem entsprechenden Anschlag an der zweiten Schnittstellenkomponente 20 an und wird über das Sicherungselement 43 gesichert. Somit liegt eine gesicherte Stellung entweder in der entriegelten oder in der verriegelten Stellung vor. Eine Zwischenstellung ist aufgrund der Formgestaltung des Sicherungselementes 43 nicht möglich. Der Vorsprung 425 an der zweiten Formschlusskomponente 42 verhindert, dass das Sicherungselement 43 vollständig in die Verriegelungsstellung zurückbewegt wird, wenn von der dargestellten Ausgangsstellung eine Verdrehung von weniger als 180° stattfindet. Die Vorderkante des Sicherungselementes 43 ist mit einer Rundung oder Formgebung ausgebildet, die der Außenkontur außerhalb des Vorsprunges 425 der zweiten Formschlusskomponente 42 entspricht, vorliegend kreisbogenförmig.

Über eine Feder 45 wird das Sicherungselement 43 in der Verriegelungsstellung gehalten oder vorgespannt, das Sicherungselement 43 ist über einen Achsbolzen 431 an der zweiten Schnittstellenkomponente 20 schwenkbar gelagert. In die Nut 423 in dem Vorsprung 425 gegenüberliegenden Ende der zweiten Formschlusskomponente 42 greift ein Sicherungsstift 424 ein, sodass eine ungewollte Bewegung in Längserstreckung oder ein Herausfallen der zweiten Formschlusskomponente 42 aus der zweiten Schnittstellenkomponente 20 nicht möglich ist.

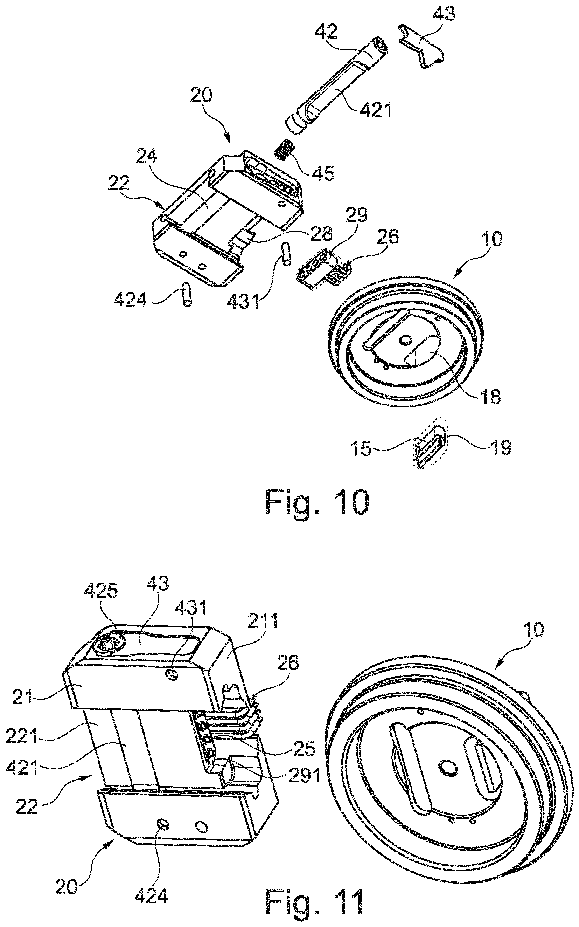

Die Figur 10 zeigt die Konfiguration gemäß Figur 9 in Untenansicht. Die Abflachung 421 ist ebenso zu erkennen wie der Verlauf der Bohrung 24 im Bereich der Nut 22. An der gegenüberliegenden Seite, also von der Stirnseite kommend ist die Steckeraufnahme 28 für den Stecker 29 zu erkennen.

In der Figur 11 ist eine Untenansicht der jeweils fertig montierten, aber noch nicht gefügten Schnittstellenkomponenten 10, 20 gezeigt. Die Abflachung 421 ist parallel oder bündig mit der Oberseite oder dem Nutgrund 221 der Nut 22 und im Wesentlichen parallel zur Grundfläche 21. Der Stift 424 verriegelt die zweite Formschlusskomponente 42 gegen ein Herausfallen und das Sicherungselement 43 liegt an dem Vorsprung 425 in der dargestellten Öffnungsstellung an und verhindert ein ungewolltes Verdrehen, sodass eine glatte und vollständige Verschiebung der beiden Schnittstellenkomponenten entlang der Schlittenführung durch die Nut 22 und Feder 12 erfolgen kann. Der Figur 11 ist die umlaufende Dichtung 291 um die elektrischen Kontakte 25 zu erkennen.

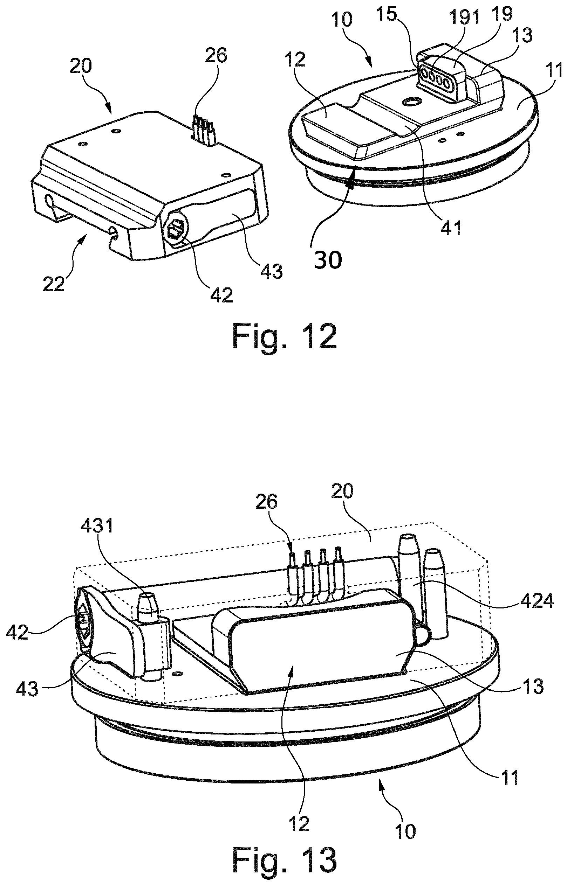

Figur 12 zeigt eine andere Darstellung des Zustandes gemäß Figur 11, der Stecker 19 in der ersten Schnittstellenkomponente 10 ist innerhalb des Endanschlages 13 eingefügt, die elektrischen Kontakte 15 sind von einer Dichtung 191 umgeben. Die erste Formschlusskomponente 41 in Gestalt der Nut oder zylindrischen oder ovalen Ausnehmung verläuft quer zur Längserstreckung der Feder 12 und im Wesentlichen parallel zur Ebene der Grundfläche 11. Die Schlittenführung 30 aus Nut 22 und Feder 12 ermöglicht das Verbinden der beiden Schnittstellenkomponenten 10, 20, indem die Feder 12 in die Schwalbenschanznut 22 eingeschoben wird.

In der teilperspektivischen Darstellung mit den durchbrochenen Linien ist der gefügte Zustand gemäß Figur 7 in der Figur 13 zu erkennen. Der Bolzen 431 stellt eine drehbare Lagerung des Sicherungselementes 43 in der zweiten Schnittstellenkomponente 20 sicher. Der zweite Bolzen 424 sichert die zweite Formschlusskomponente 42 gegen eine translatorische Bewegung. In der dargestellten Position ist noch keine formschlüssige Verriegelung der Schnittstellenanordnung erfolgt, dazu muss die zweite Formschlusskomponente 42 um 180° gedreht werden, nachdem das Sicherungselement 43 außer Eingriff gebracht worden ist.

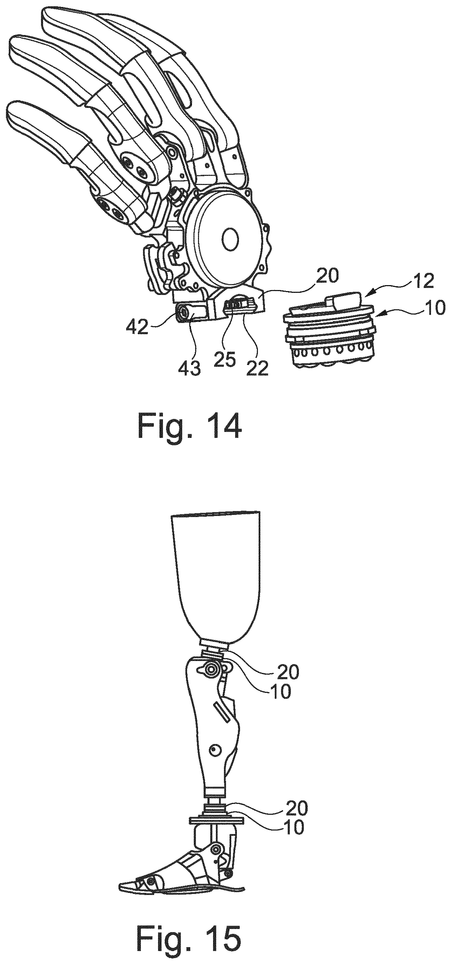

In der Figur 14 ist ein Anwendungsbeispiel für die Schnittstellenanordnung an einer Prothesenhand gezeigt. Die Prothesenhand mit den Prothesenfingern und einem Antrieb ist an der zweiten Schnittstellenkomponente 20 angeordnet, an dem proximalen Ende ist die Nut 22 eingearbeitet. Die entsprechenden elektrischen Kontakte 25 sind an dem nicht mehr zu erkennenden Stecker in der Nut 22 angeordnet. Die erste Schnittstellenkomponente 10 ist als distales Endstück an einem Drehadapter angeordnet und wird an einem Prothesenschaft oder einem Unterarmrohr angeordnet.

Eine elektrische Kontaktierung und Durchleitung von Energie und Daten findet über die elektrischen Kontakte 25 und 15 statt, die an dem Endanschlag der Feder 12 ausgebildet oder angeordnet sind.

Weitere Anwendungsbeispiele sind in der Figur 15 dargestellt, bei der die Schnittstellenanordnung einmal zwischen einem Oberschenkelschaft und einem Prothesenkniegelenk sowie einem Unterschenkelrohr und einem Prothesenfuß ausgebildet sind.

Über die Schnittstellenanordnung finden neben einer sicheren, formschlüssigen Befestigung der jeweiligen Komponenten aneinander auch eine elektrische Kontaktierung sowie eine Abschirmung der elektrischen Kontaktierung gegen Verschmutzung und Feuchtigkeit statt. Es wird eine knickstabile, sichere Kontaktierung bei einer gleichzeitigen mechanischen Verriegelung bereitgestellt.

## Patentansprüche

1. Schnittstellenanordnung mit einer ersten Schnittstellenkomponente (10) und einer zweiten Schnittstellenkomponente (20) mit einer Schlittenführung (30) zur mechanischen Festlegung der Schnittstellenkomponenten (10, 20) aneinander, wobei an einer der Schnittstellenkomponenten (10) eine von einer Grundfläche (11) hervorstehende Feder (12) und an der anderen Schnittstellenkomponente (20) eine in einer Grundfläche (21) liegende Nut (22) mit korrespondierenden Querschnitten angeordnet sind, wobei die Feder (12) und die Nut (22) die Schnittstellenkomponenten (10, 20) aneinander formschlüssig festlegen und eine Bewegung senkrecht zu den Grundflächen und senkrecht zu der Längserstreckung der Feder (12) und Nut (22) sperren, **wobei** an der Feder (12) und in der Nut (22) elektrische Kontaktelemente (15, 25) angeordnet sind, die in einem gefügten Zustand der Schnittstellenkomponenten (10, 20) eine elektrische Verbindung herstellen, **dadurch gekennzeichnet, dass** die Nut (22) in dem gefügten Zustand mit der Feder (12) über eine stirnseitige Dichtung (191, 291) abgedichtet ist und dass die Schnittstellenkomponenten (10, 20) eine Verriegelungseinrichtung mit zwei wiederholbar in und außer Eingriff miteinander bringbaren Formschlusskomponenten (41, 42) aufweisen, die eine Verlagerung der Schnittstellenkomponenten (10, 20) zueinander entlang der Längserstreckung der Schlittenführung (30) sperrt, wobei die Formschlusskomponente (42) drehbar oder schwenkbar gelagert und zumindest eine Abflachung (421), einen unrunden Querschnitt oder Ausnehmung aufweist und in der Verriegelungsstellung die Schnittstellenkomponenten (10, 20) gegeneinander verspannt.

2. Schnittstellenanordnung nach Anspruch 1, **dadurch gekennzeichnet, dass** die elektrischen Kontaktelemente (15, 25) an einer Stirnseite eines Endanschlages (13) der Feder (12) und/oder an einer Stirnseite eines Endanschlages (23) der Nut (22) angeordnet sind.

3. Schnittstellenanordnung nach Anspruch 2, **dadurch gekennzeichnet, dass** der Endanschlag (13, 23) eine Anlagefläche für einen Stecker (19, 29) oder eine Buchse mit den Kontaktelementen (15, 25) ausbildet.

4. Schnittstellenanordnung nach einem der voranstehenden Ansprüche, **dadurch gekennzeichnet, dass** eine Kabelverbindung (16, 26) von den elektrischen Kontaktelementen (15, 25) von der Feder (12) und der Nut (22) wegführend verlegt sind.

5. Schnittstellenanordnung nach einem der voranstehenden Ansprüche, **dadurch gekennzeichnet, dass** in zumindest einer Schnittstellenkomponente (10, 20) eine Kabeldurchführung (27) und/oder eine Steckeraufnahme (18, 28) angeordnet oder ausgebildet ist.

6. Schnittstellenanordnung nach einem der voranstehenden Ansprüche, **dadurch gekennzeichnet, dass** einer Formschlusskomponente (42) ein Sicherungselement (43) zugeordnet ist, das von einer Stirnseite (211) der Schnittstellenkomponente (20) zugänglich ist.

7. Schnittstellenanordnung nach Anspruch 6, **dadurch gekennzeichnet, dass** das Sicherungselement (43) formschlüssig in die Formschlusskomponente (42) eingreift, gegen das Formschlusselement (42) vorgespannt ist und/oder über eine Verzahnung (432) mit dem Formschlusselement (42) gekoppelt ist.

8. Schnittstellenanordnung nach einem der voranstehenden Ansprüche, **dadurch gekennzeichnet, dass** eine Formschlusskomponente (41) als Nut ausgebildet ist.

9. Schnittstellenanordnung nach einem der voranstehenden Ansprüche, **dadurch gekennzeichnet, dass** die erste Schnittstellenkomponente (10) als distale Abschlussplatte eines Prothesenschaftes oder einer Prothesenkomponente ausgebildet ist.

10. Schnittstellenanordnung nach einem der voranstehenden Ansprüche, **dadurch gekennzeichnet, dass** die zweite Schnittstellenkomponente (20) als proximale Abschlusskomponente einer Prothesenhand oder einer prothetischen Greifeinrichtung ausgebildet ist.

## Claims

1. An interface assembly with a first interface component (10) and a second interface component (20) with a sliding guide (30) for mechanically fixing the interface components (10, 20) to one another, wherein a tongue (12) projecting from a base surface (11) is arranged on one of the interface components (10) and a groove (22) with corresponding cross sections located in a base surface (21) is arranged on the other interface component (20), wherein the tongue (12) and the groove (22) lock the interface components (10, 20) to each other in a form-fitting manner and block a movement perpendicular to the base surfaces and perpendicular to the longitudinal extension of the tongue (12) and groove (22), wherein electrical contact elements (15, 25) are arranged on the tongue (12) and in the groove (22), said contact elements establishing an electrical connection in a joined state of the interface components (10, 20), **characterized in that** the groove (22) is sealed by a seal (191, 291) on the end face in the joined state with the tongue (12) and **in that** the interface components (10, 20) have a locking device (41, 42) with two form-fitting components (41, 42) which can be repeatedly engaged and disengaged with each other which blocks displacement of interface components (10, 20) relative to one another along the longitudinal extent of the sliding guide (30), wherein the form-fitting component (42) is rotatably or pivotally mounted and has at least one flattening (421), non-circular cross-section or recess and braces the interface components (10, 20) against each other in the locked position.

2. The interface assembly according to claim 1, **characterized in that** the electrical contact elements (15, 25) are arranged on an end face of an end stop (13) of the tongue (12) and/or on an end face of an end stop (23) of the groove (22).

3. The interface assembly according to claim 2, **characterized in that** the end stop (13, 23) forms a contact surface for a plug (19, 29) or a socket with the contact elements (15, 25).

4. The interface assembly according to one of the preceding claims, **characterized in that** a cable connection (16, 26) is laid by the electrical contact elements (15, 25) leading away from the tongue (12) and the groove (22).

5. The interface assembly according to one of the preceding claims, **characterized in that** a cable duct (27) and/or a plug receiver (18, 28) is arranged or formed in at least one interface component (10, 20).

6. The interface assembly according to one of the preceding claims, **characterized in that** a form-fitting component (42) is assigned a securing element (43) which is accessible from an end face (211) of the interface component (20).

7. The interface assembly according to claim 6, **characterized in that** the securing element (43) engages in the form-fitting component (42) in a form-fitting manner, is preloaded against the form-fitting component (42) and/or is coupled to the form-fitting component (42) via a toothing (432).

8. The interface assembly according to one of the preceding claims, **characterized in that** a form-fitting component (41) is designed as a groove.

9. The interface assembly according to one of the preceding claims, **characterized in that** the first interface component (10) is designed as a distal end plate of a prosthesis socket or a prosthesis component.

10. The interface assembly according to one of the preceding claims, **characterized in that** the second interface component (20) is formed as a proximal end component of a prosthetic hand or a prosthetic gripping device.

## Revendications

1. Ensemble formant interface, comprenant un premier composant d'interface (10) et un deuxième composant d'interface (20) muni d'un guidage à chariot (30) destiné à fixer mécaniquement les composants d'interface (10, 20) l'un à l'autre, dans lequel
une languette (12) faisant saillie à partir d'une surface de base (11) est disposée sur l'un des composants d'interface (10), et une rainure (22) située dans une surface de base (21) est disposée sur l'autre composant d'interface (20), lesquelles présentant des sections transversales qui se correspondent,
la languette (12) et la rainure (22) fixent les composants d'interface (10, 20) l'un à l'autre par complémentarité de forme et empêchent tout mouvement perpendiculaire aux surfaces de base et perpendiculaire à l'extension longitudinale de la languette (12) et de la rainure (22),
des éléments de contact électriques (15, 25) sont disposés sur la languette (12) et dans la rainure (22), qui établissent une connexion électrique lorsque les composants d'interface (10, 20) sont assemblés,
**caractérisé en ce que**, lorsque la rainure (22) est assemblée avec la languette (12), elle est rendue étanche au moyen d'un joint d'étanchéité frontal (191, 291), et **en ce que** les composants d'interface (10, 20) comportent un dispositif de verrouillage qui comprend deux composants de liaison par complémentarité de forme (41, 42), aptes à être mis en prise et hors de prise de manière répétée, et qui empêche tout déplacement des composants d'interface (10, 20) l'un par rapport à l'autre le long de l'extension longitudinale du guidage à chariot (30),
sachant que le composant de liaison par complémentarité de forme (42) est monté de manière à pouvoir tourner ou pivoter, présente au moins un méplat (421), une section transversale non circulaire ou un évidement, et, en position de verrouillage, vient serrer les composants d'interface (10, 20) l'un contre l'autre.

2. Ensemble formant interface selon la revendication 1,
**caractérisé en ce que** les éléments de contact électriques (15, 25) sont disposés sur une face frontale d'une butée de fin de course (13) de la languette (12) et/ou sur une face frontale d'une butée de fin de course (23) de la rainure (22).

3. Ensemble formant interface selon la revendication 2,
**caractérisé en ce que** la butée de fin de course (13, 23) constitue une surface d'appui pour une fiche (19, 29) ou pour une douille comportant les éléments de contact (15, 25).

4. Ensemble formant interface selon l'une des revendications précédentes, **caractérisé en ce qu'**une connexion par câble (16, 26) part des éléments de contact électriques (15, 25) en s'éloignant de la languette (12) et de la rainure (22).

5. Ensemble formant interface selon l'une des revendications précédentes, **caractérisé en ce qu'**un passage de câble (27) et/ou un logement de fiche (18, 28) est agencé ou formé dans au moins un composant d'interface (10, 20).

6. Ensemble formant interface selon l'une des revendications précédentes, **caractérisé en ce qu'**un élément de blocage (43) est associé à un composant de liaison par complémentarité de forme (42), lequel élément est accessible depuis une face frontale (211) du composant d'interface (20).

7. Ensemble formant interface selon la revendication 6,
**caractérisé en ce que** l'élément de blocage (43) s'engage par complémentarité de forme dans le composant de liaison par complémentarité de forme (42), est précontraint contre l'élément de liaison par complémentarité de forme (42) et/ou est couplé à l'élément de liaison par complémentarité de forme (42) par l'intermédiaire d'une denture (432).

8. Ensemble formant interface selon l'une des revendications précédentes, **caractérisé en ce qu'**un composant de liaison par complémentarité de forme (41) est réalisé sous la forme d'une rainure.

9. Ensemble formant interface selon l'une des revendications précédentes, **caractérisé en ce que** le premier composant d'interface (10) est réalisé sous la forme d'une plaque de terminaison distale d'une emboîture de prothèse ou d'un composant de prothèse.

10. Ensemble formant interface selon l'une des revendications précédentes, **caractérisé en ce que** le deuxième composant d'interface (20) est réalisé sous la forme d'un composant de terminaison proximal d'une prothèse de main ou d'un dispositif de préhension prothétique.
